# EUROPEAN PATENT APPLICATION

(11) **EP 1 181 936 A1**
(43) Date of publication of application: **27.02.2002**
(21) Application number: 01600010.1
(22) Date of filing: 18.05.2001
(51) Int. Cl.: A61K 38/10, C07K 7/08, A61K 51/08, C07F 15/00

(54) **Synthesis, biological and preclinical evaluation of (D-Phe6,Leu-NHEt13,des-Met14)bombesin(6-14) analogs modified with 1,4,8,11-tetraazaundecane derivatives and labeled with technetium-99m for application in oncology**

(30) Priority: 25.05.2000 GR 2000100175
(71) Applicant: N.C.S.R. "DEMOKRITOS"-Institute of Radioisotopes & Radiodiagnostic Products, 153 10 Aghia Paraskeyi Attikis (GR)
(72) Inventor: Chiotellis, Efstratios, Ph.D., 11635 Athens (GR); Nock, Berthold, Ph.D., 15233 Halandri, Athens (GR); Maina, Theodosia, Ph.D., 15233 Halandri, Athens (GR)

(57) **Abstract**

In the present invention the synthesis of analogs of the bombesin (BBN) antagonist, (D-Phe⁶,Leu-NHEt¹³,des-Met¹⁴)BBN(6-14), is described, to which 1,4,8,11-tetraazaundecane derivatives have been coupled, in order to enable labeling with technetium-99m aiming at their application in the in vivo localization of BBN/GRP-positive neoplasms in the human applying SPECT (single photon emission computed tomography).

In addition, the method of labeling of (D-Phe⁶,Leu-NHEt¹³,des-Met¹⁴)BBN(6-14) analogs, modified with 1,4,8,11-tetraazaundecane derivatives, with technetium-99m is described.

This invention also includes the radiochemical evaluation of technetium-99m complexes deriving from the above steps: labeling yield, lipophilicity measurement and in vitro stability.

Finally this invention describes also the preclinical evaluation of the analogs deriving from the above steps:
- In cell lines overesxpressing the BBN/GRP receptor
- in in vivo animal models
and the study of their metabolism.

## Description

### Technical field

Subjects of this invention are methods, compounds, syntheses as well as the biological and preclinical evaluation of (D-Phe⁶,Leu-NHEt¹³,des-Met¹⁴)-Bombesin(6-14) ((D-Phe⁶,Leu-NHEt¹³,des-Met¹⁴)BBN(6-14)) analogs modified with 1,4,8,11-tetraazaundecane derivatives and labeled with technetium-99m, aiming at their application in Oncology.

Subject of the invention is the synthesis of (D-Phe⁶,Leu-NHEt¹³,des-Met¹⁴)-BBN(6-14) analogs modified with 1,4,8,11-tetraazaundecane derivatives.

Another subject of the invention are methods of labeling of (D-Phe⁶,Leu-NHEt¹³,des-Met¹⁴)BBN(6-14) analogs modified with 1,4,8,11-tetraazaundecane derivatives with technetium-99m.

Another subject of the invention is the radiochemical evaluation of the technetium complexes that form from the previous steps: labeling yields, lipophilicity estimation and in vitro stability.

Another subject of the invention is the biological evaluation of the analogs that form from the previous steps:
- in cell lines that overexpress the BBN/GRP receptor
- in in vivo healthy and human tumor bearing animal models
and the study of their metabolism.

### Disclosure of the invention

### State of the art

Metastable technetium-99m (^{99m}Tc) is the gold standard of Nuclear Medicine. More than 80% of diagnostic radiopharmaceuticals currently used for clinical studies are ^{99m}Tc based compounds, utilized in the imaging of the morphology and pathophysiology of organs and tissues of the human body.
The widespread use of ^{99m}Tc is a result of its almost ideal nuclear properties. The rather short half-life (t_{1/2}: 6 h) along with the absence of particle emission of ^{99m}Tc minimize the radiation dose to the patient. Its monoenergetic Y-radiation (140 keV) is considered very efficient for imaging applying today's Nuclear Medicine instrumentation. In addition to these advantages, ^{99m}Tc is cost effective and widely distributed (in long distances from its production site), through commercial ⁹⁹Mo - 99mTc generators. ^{99m}Tc is eluted from the ⁹⁹Mo - ^{99m}Tc generator in the form of a sterile solution of sodium pertechnetate (^{99m}TcO₄Na) in physiological saline.

For the clinical application, ^{99m}Tc must be coordinated forming ^{99m}Tc complexes, that will direct the metal radionuclide to the desired target tissue (cancer cell) and lead to the required pharmacokinetics in the human body.

Recent advances in Oncology involve the use of peptide analogs labeled with diagnostic or therapeutic radionuclides in the in vivo localization or/and therapy of neoplasms. This approach is based on the fact that many types of neoplastic cells overexpress on their cell membrane peptide receptors, as for example receptors of the peptide hormone somatostatin (SMS). In this respect the in vivo imaging of tumors and their metastases is attempted today by SPECT (single photon emission computed tomography) and the use of peptide analogs labeled with the radionuclides ^{99m}Tc, ⁶⁷Ga, ¹¹¹In, ¹²³I etc, or alternatively by PET (positron emission tomography) and the use of peptide analogs labeled with the radionuclides ⁶⁴Cu, ⁶⁸Ga, ¹⁸F etc. At the same time many efforts have been focused on the use of peptide analogs labeled with the radionuclides ⁶⁴Cu, ⁶⁷Cu, ¹¹¹In, ⁹⁰Y, ¹⁸⁸Re, for radiotherapy, which have not so far led to the development of an approved and commercially available radiotherapeutic agent.

Bombesin (BBN) is a natural tetradecapeptide first isolated from the skin of the european frog *Bombina bombina* [1]. It has been demonstrated that BBN and its related peptides, gastrin releasing peptide (GRP), neuromedin B (NMB B) and neuromedin C (NMB C, GRP 18-27), participate in a wide spectrum of biological processes in various mammal tissues. Such actions include the regulation of central nervous system (CNS) functions, like thermoregulation [2], activation of enzyme release from exocrine glands [3], activation of gastroenteropancreatic (GEP) peptide hormone release [4] etc. It has also been reported, that these peptides may act as autocrine growth factors in normal and cancer tissues, as for example, in the case of small cell lung cancer (SCLC) [5].

The action of BBN is manifested after its binding to specific receptors (BBN/GRP receptors), located on the cell membrane of target cells, most of which belong to the superfamily of G-protein coupled receptors [6].

The overexpression of BBN/GRP receptors in certain types of cancer, as for example pancreatic carcinomas, colon carcinomas, prostate cancer (PC) , or small cell lung cancer (SCLC), renders this biomolecule appropriate for the in vivo imaging of BBN/GRP positive tumors and their metastases [7]. According to this approach, BBN and its analogs direct the localization of radionuclides suitable either for diagnostic or therapeutic purposes specifically onto BBN/GRP positive cancer cells.

In addition, the small size of BBN leads to a very favorable pharmacokinetic profile, characterized by a rapid localization in in vivo binding sites and a fast blood clearance, characteristics very promising for applications in Nuclear Medicine.

From the above it can be concluded, that the in vivo imaging of BBN/GRP positive tumors and their metastases by means of labeled peptide analogs of BBN is promising.

Given that the majority of the above applications involves metallic radionuclides, the parent bioactive compound must be suitably modified, in order to guarantee both the efficient binding of the radiometal and the preservation of biological activity of the forming radioligand. This is usually achieved by covalently coupling of the appropriate bifunctional chelator to a selected site of the parent compound. The selection of the most efficient bifunctional chelator for each metallic radionuclide along with the position of coupling on the parent compound play a key role in the success of this method.

### Advantages of the invention versus currently applied methods

Given that metastable ^{99m}Tc remains the main diagnostic radionuclide in Nuclear Medicine [8,9], we synthesized and characterized two novel BBN analogs labeled with ^{99m}Tc, BBN-1 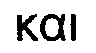 BBN-2, which exhibit affinity and selectivity for the BBN/GRP receptor.

The known BBN antagonist (D-Phe⁶,Leu-NHEt¹³,des-Met¹⁴)BBN(6-14) [10] has been used as the parent compound, to which the pertinent 1,4,8,11-tetraazaundecane analogs were covalently coupled. This synthesis was performed in solution. The products were characterized by HPLC, UV/Vis and ES-MS spectroscopies.

Labeling with ^{99m}Tc is quantitative and leads to a stable labeled product of high specific activity.

These analogs showed a high affinity binding to the BBN/GRP receptor in competitive binding experiments in membrane homogenates of PC-3 cells, which overexpress the BBN/GRP receptor.

The study of their biodistribution in healthy mice revealed their rapid clearance from the body of mice into the urine through the kidneys and the urinary system.

In vivo blocking experiments demonstrated that the radioactivity accumulation in the pancreas, but also in the intestines, the main BBN/GRP receptor rich organs, is specific, while non-specific radioactivity accumulation in any other organ or tissue was not observed.

Moreover, according to the metabolism study findings these, while stable in the murine plasma, are rapidly degraded in the main excretory organs (the kidneys and the liver) and are excreted in the urine totally converted into two hydrophilic metabolites.

Further study of the above two analogs in the human has been scheduled.

### Detailed description of the invention

### EXPERIMENTAL

### -Synthesis of the peptide analogs

The synthesis of the peptide analogs was achieved by coupling of the 1,4,8,11-tetraazaundecane derivatives to the N-terminal amino acid of (D-Phe⁶,Leu-NHEt¹³,des-Met¹⁴)BBN(6-14) in solution following BOC protection strategies, as shown in Scheme 1 (Synthesis of BBN-1 (**A**) and BBN-2 (**B**) applying BOC methodology). The formation and purity of the two products was confirmed by high performance liquid chromatography (HPLC) and electrospray mass spectroscopy (ES-MS).

### -Labeling of BBN analogs with ^{99m}Tc

To Na^{99m}TcO₄ (730 µL) generator eluate 0.5 M pH 11.5 (200 µL) phosphate buffer was added followed by a 0.1 M (10 µL) citrate solution, an aqueous solution of the respective BBN analog (10 nmol, 40 µL) and a fresh solution of SnCl₂ in EtOH (20 µL, 20 µg). The labeling reaction mixture was incubated for 30 min at room temperature and then the pH was adjusted to 7.0 by addition of 1 M HCI. The labeling yield was tested by HPLC analysis. The formulae of the two labeled products together with their respective radiochromatograms are depicted in Scheme 2 (^{99m}Tc labeled BBN-1 (**A**) and ^{99m}Tc labeled BBN-2 (**B**) with their respective HPLC radiochromatograms).

### - In vitro competition binding assays of BBN analogs to the BBN/GRP receptor

Competition binding assays of the two BBN analogs (BBN-1 and BBN-2) as well as of Tyr⁴-BBN (control) were performed in cell membrane homogenates of the PC-3 cell line using ¹²⁵I-Tyr⁴-BBN as the radioligand. The PC-3 cell line overexpresses the human GRP receptor and originates from human prostate carcinoma. Briefly, 40000 cpm of radioligand were used per assay tube, with 50 µg protein in the presence of increasing concentration of tested hormone in a total volume of 300 µL 50 mM HEPES (pH 7.6, 0.3% BSA, 5 mM MgCl₂, 14 mM Bacitracin). Samples were incubated for 30 min at 37°C and then diluted with 10 mL chilled buffer (10 mM HEPES, 150 mM NaCI, pH 7.6). The suspension was rapidly filtered through suitable filters (glass fibre filters Whatman GF/B, presoaked in 0.3% BSA) in a semi-automatic apparatus (Brandel Cell Harvester). Filter radioactivity was measured on an automated γ counter of Nal(TI) well type. As non-specific binding was characterized the amount of radioactivity that was bound in the presence of 1 µM Tyr⁴-BBN.

### -Biodistribution experiments of ^{99m}Tc labeled BBN analogs in healthy mice

For biodistribution experiments Swiss albino mice (30 ± 5 g) were used in groups of four. In each animal a solution of radiolabeled peptide in PBS buffer pH 7.4 (100 µL, 2.5 - 4 mCi) was administered through the tail vein. Animals were sacrificed by cardiac puncture at predetermined time intervals (30 min, 1, 2 and 4 h postinjection, pi). Samples of urine, blood as well as organs of interest were collected immediately, weighed and their radioactivity content was measured in a γ-counter.
In an additional group of animals 1 mg unlabeled peptide was administered intraperitoneally (ip) 35 min prior to the injection of the radioligand (blocked animals). These animals were sacrificed 30 min after the radioligand intravenous (iv) injection.
Biodistribution data were calculated as percent injected dose per organ (% ID/organ) and as percent injected dose per gram tissue (% ID/gr) according to a known algorithm.

### -Metabolism experiments

The urine collected within the first 30 min after injection of the radioligand was analyzed by HPLC.
Murine plasma isolated by centrifugation of whole blood for 15 min at 5000 rpm was incubated with radiolabeled analogs at 37°C for 1 h and samples at predetermined time intervals were analyzed by HPLC.
Kidneys or liver freshly excised were rinsed and placed in a chilled 50 mM Tris, 0.2 M sucrose pH 7.4 buffer, where they were homogenized in an Ultra-Turrax T25 homogenator for 30 s at 4°C. These homogenates were also incubated with labeled analogs at 37°C and aliquots of 5 and 30 min incubates analyzed by HPLC.

### -Biodistribution experiments of ^{99m}Tc labeled BBN analogs in PC-3 tumor bearing mice

For biodistribution experiments adult female athymic swiss nu/nu mice (6 weeks of age) were used. The animals received subcutaneously in their flanks a suspension containing 1 - 2 x 10⁷ PC-3 cells and 2 - 3 weeks later tumors of adequate size have developed. For the tissue distribution experiment groups of four animals per time point (1 and 4 h pi) were used. Two parallel groups of animals were injected iv 250 µg Tyr⁴-BBN together with the radioligand (blocked animals). The experiment was further performed as described above.

### RESULTS - DISCUSSION

### -Synthesis of peptide analogs

The respective BOC-protected 1,4,8,11-tetraazaundecane derivative was coupled to the terminal amino group of the peptide chain of the BBN antagonist (D-Phe⁶,Leu-NHEt¹³,des-Met¹⁴)BBN(6-14), either through a benzylamino diglycolic acid spacer (BBN-1) or directly, through a carboxy group (BBN-2). The above synthesis was achieved in solution in the presence of HATU and Hünig's base. The BOC-protected conjugates were purified, deprotected by TFA addition and isolated by preparative HPLC (Scheme 1). The ES-MS spectra confirmed the formation of expected compounds.

### -Labeling of BBN analogs with ^{99m}Tc

Labeling with ^{99m}Tc was performed in phosphate buffer pH 11.5 using citrate as transfer ligand and SnCl₂ for the reduction of pertechnetate. As demonstrated by HPLC analysis of the labeled product, labeling was practically quantitative (>95 %) in high specific activities, even when using peptide amounts as low as 10 nmol. In the representative radiochromatograms of Scheme 2 it is clear that the ^{99m}Tc-BBN-1 analog is, as expected by the presence of an additional phenyl ring, less hydrophilic than ^{99m}Tc-BBN -2.

### -In vitro binding assays of BBN analogs to the BBN/GRP receptor

The binding capabilities of BBN-1 and BBN-2 analogs to the BBN/GRP receptor were measured by appropriate competition binding experiments in PC-3 cell membrane preparations using ¹²⁵I-Tyr⁴-BBN as the radioligand and Tyr⁴-BBN as control. Both products demonstrated high affinity binding to the BBN/GRP receptor with IC₅₀ values in the subnanomolar range, as shown in Scheme 3 (Competition binding of ¹²⁵I-Try⁴-BBN to GRP/BBN receptors in PC-3 cell membrane preparations in the presence of increasing concentrations of BBN-1 and BBN-2 analogs).

### -Biodistribution experiments of ^{99m}Tc labeled BBN analogs in healthy mice

The labeled peptide analogs were administered via the tail vein in Swiss albino mice, sacrificed at 30 min, 1, 2 and 4 h intervals pi. Biodistribution results are presented as percent injected dose per gram tissue (%ID/g) in Scheme 4 (Biodistribution data of ^{99m}Tc BBN-1 (**A**) and ^{99m}Tc BBN-2 (**B**) in healthy mice in %ID/g; Bl.= blood, Li.= liver, He.= heart, Ki.= kidneys, St.= stomach, In.= intestines, Sp.= spleen, Mu.= muscle, Lu.= lungs, Pa.= pancreas). Both analogs are rapidly washed out from the body of mice into the urine. The ^{99m}Tc-BBN-2 analog is excreted faster, while ^{99m}Tc-BBN-1 shows a somewhat higher uptake in the liver and the intestines. The high pancreas uptake of ^{99m}Tc-BBN-1 at 30 min pi (the critical organ for the BBN receptor) was totally blocked in the animals that were pretreated with a high dose of unlabeled peptide (in vivo blocking experiments). In vivo blocking for both analogs was observed to a lesser extent also in the intestines, given that BBN receptors in a lower density are located throughout the gastrointestinal tract.
The above properties result in high target to non-target tissue ratios, as summarized in Scheme 5 (Important target to non-target tissue ratios in the biodistribution of ^{99m}Tc BBN-1 and ^{99m}Tc BBN-2 in healthy mice (P/BI= pancreas to blood, P/Li= pancreas to liver, P/Mu= pancreas to muscle and P/BP= pancreas to blocked pancreas).

### -Metabolism experiments

The HPLC analysis of urine collected in the first 30 min after injection of the two labeled analogs revealed that none of them remains intact in vivo, but they are both excreted in the form of two hydrophilic metabolites, whose structure in under investigation.
The above analogs, as revealed by the HPLC analysis of 1 h incubates in murine plasma at 37°C, are stable in plasma. Incubation of the labeled analogs with kidney or liver homogenates at 37°C, demonstrated that both analogs are rapidly degraded to hydrophilic products in the kidney and to a slower rate also in the liver (Table 1).

**TABLE 1:**

| In vivo metabolic study of ^{99m}Tc-labeled peptide analogs.* | | | | | | | |
|---|---|---|---|---|---|---|---|
| | URINE | PLASMA | KIDNEYS | | | LIVER | |
| | 30 min | 1 h | 1 min | 5 min | 30 min | 5 min | 30 min |
| BBN-1 | 0% | 100% | - | 4% | 2% | 100% | 23% |
| BBN-2 | 0% | 100% | 85% | 3% | 1,5% | 95% | 30% |

| | | | | | | | |
|---|---|---|---|---|---|---|---|
| *Data represent the percent of intact labeled analog | | | | | | | |

### -Biodistribution experiments of ^{99m}Tc labeled BBN analogs in PC-3 tumor bearing mice

Tissue distribution data for ^{99m}Tc BBN-1 as percent injected dose per gram tissue (%ID/g) are summarized in Scheme 6 (Tissue distribution data as %ID/g for ^{99m}Tc BBN-1 in PC-3 tumor bearing mice; BI.= blood, Li.= liver, He.= heart, Ki.= kidneys, St.= stomach, In.= intestines, Sp.= spleen, Mu.= muscle, Lu.= lungs, Pa.= pancreas, Tu.= tumor). Besides the very favorable biokinetic profile ^{99m}Tc BBN-1 localizes very rapidly and in a high percentage in the experimental tumor, where it remains up to 4 h pi tested in this experiment. This localization is receptor-specific, given that in the blocked animals it appears reduced by more than 90%.
The above biological properties lead to high target to background ratios, as detailed in Scheme 7 (Important target to non-target tissue ratios in the biodistribution of ^{99m}Tc BBN-1 in PC-3 tumor bearing mice; T/BI= tumor to blood, T/Li= tumor to liver, T/Ki= tumor to kidneys, T/BT= tumor to blocked tumor and T/Mu= tumor to muscle).

### CONCLUSIONS

Two novel BBN analogs were synthesized and characterized. Both analogs can be labeled with ^{99m}Tc under mild conditions, in high yields and high specific activities.
The labeled compounds are hydrophilic and show a rapid wash out from the body of mice into the urine, mainly through the kidneys and the urinary tract.
Both labeled analogs accumulate in the pancreas as a result of their specific interaction with BBN receptors, as demonstrated by in vivo blocking experiments.
Both labeled analogs are stable in the plasma and are rapidly metabolized in the major excretory organs, the kidneys and the liver, without any non-specific activity accumulation in the rest of the body.
The two analogs localize in the implanted tumors rapidly and with a high specificity and remain there for sufficient time thereby leading to high tumor to background ratios.
The above favorable characteristics validate the novel ^{99m}Tc BBN-1 and ^{99m}Tc BBN-2 analogs for the scintigraphic detection of BBN/GRP-positive neoplasms in the human.

### REFERENCES

1. Anastasi A, Erspamer V, Bucci M. *Experientia* **1971**; *27*: 166
2. Marki W, Brown M, Rivier JE. *Peptides* **1981**; *2*: 169-177
3. Jensen RT, Coy DH et al. *Ann N Y Acad Sci* **1988**; *547*: 138-149
4. Ghatei MA, Jung RT et al. *J Clin Endocrinol Metab* **1982**; *54*: 980-985
5. Cuttita F, Carney DN, et al. *Nature* **1985**; *316*: 823-826
6. Burbach JPH, Meijer OC. *Eur J Pharmacol* **1992**; *227*: 1-18
7. Reubi JC. *Endocrinol Metab Clin North Am* **1993**; *22*: 917-939
8. Maina T, Stolz B, Albert R, Bruns C, Koch P, Maecke H. *Eur J Nucl Med* **1994**; *21*:437-444
9. Maina T, Stolz B, Albert R, Nock B, Bruns C, Mäcke H. In Nicolini M, Bandoli G, Mazzi U, eds. *Technetium and rhenium in chemistry and nuclear medicine* ***4***. Padova: SG Editorali; **1995**: 395-400
10.Wang LH, Coy DH, Taylor JE et al. *J Biol Chem* **1990**; *265*: 15695-15703

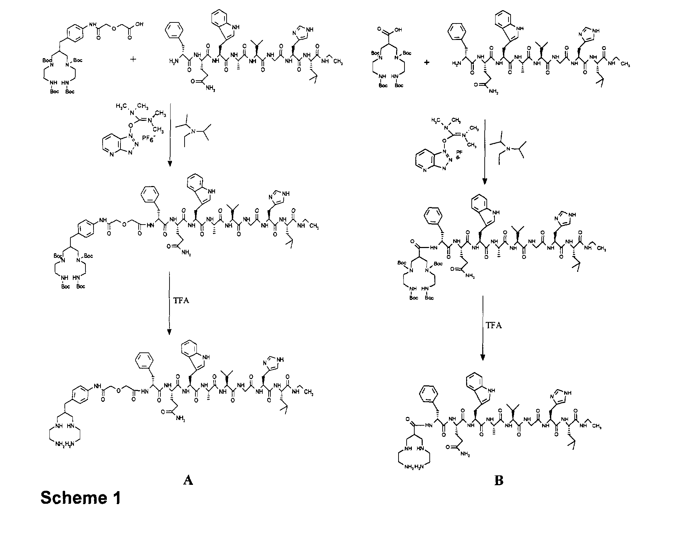

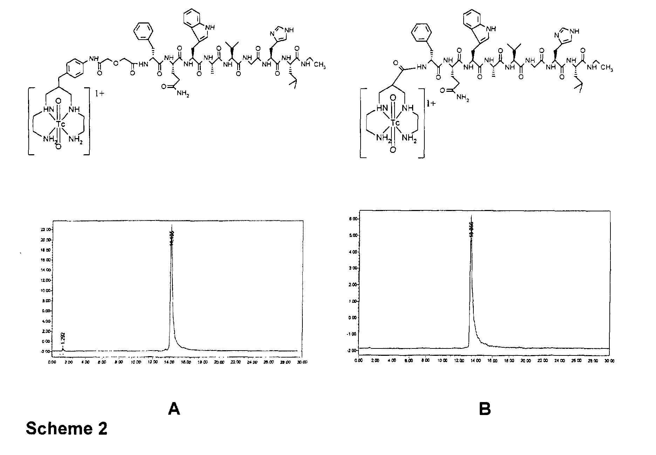

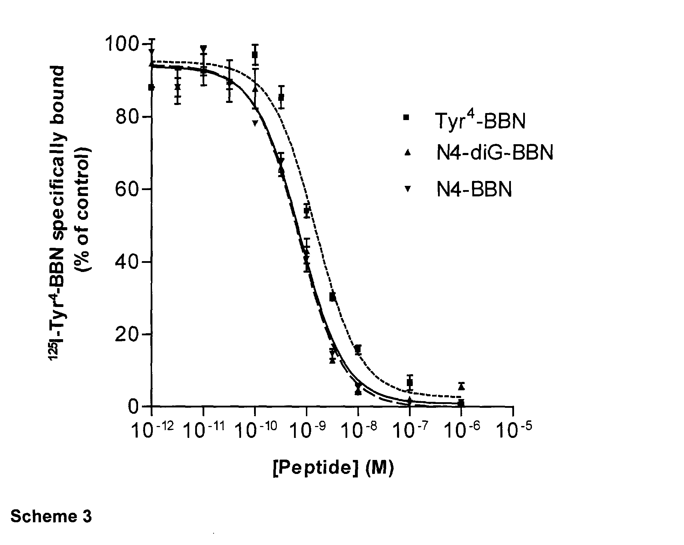

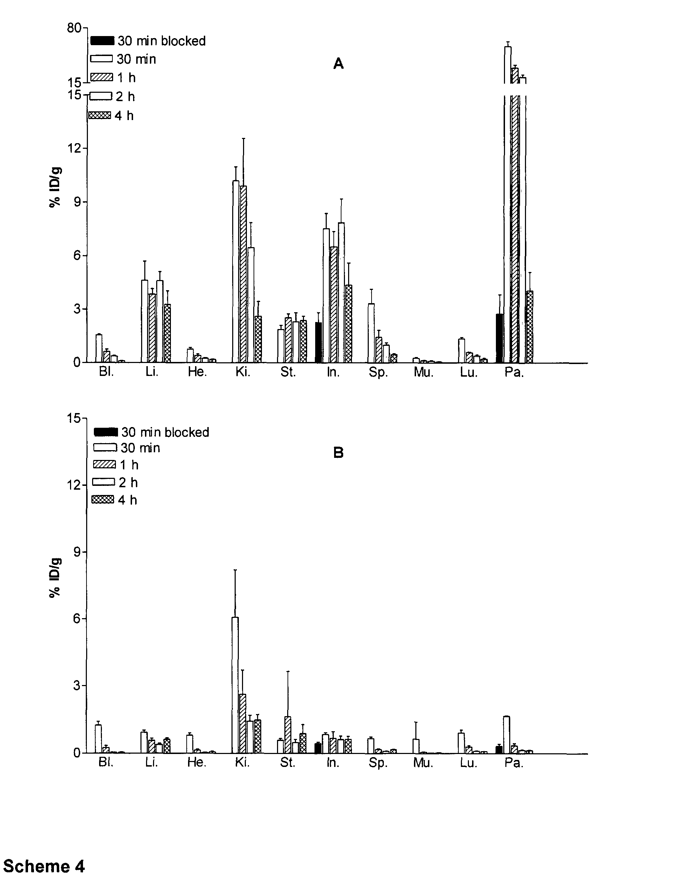

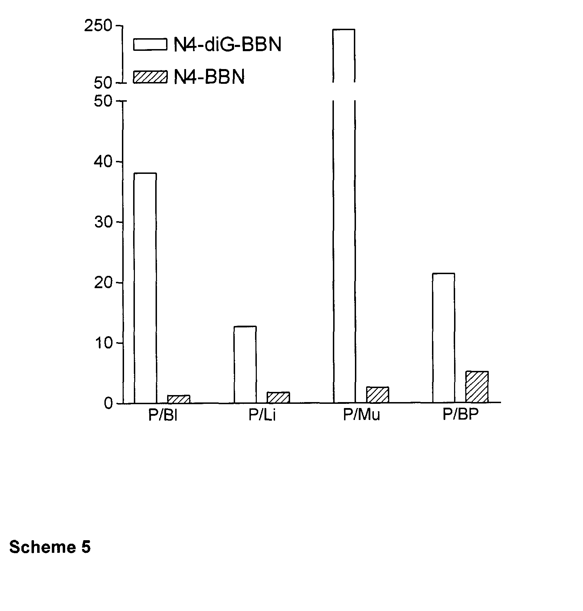

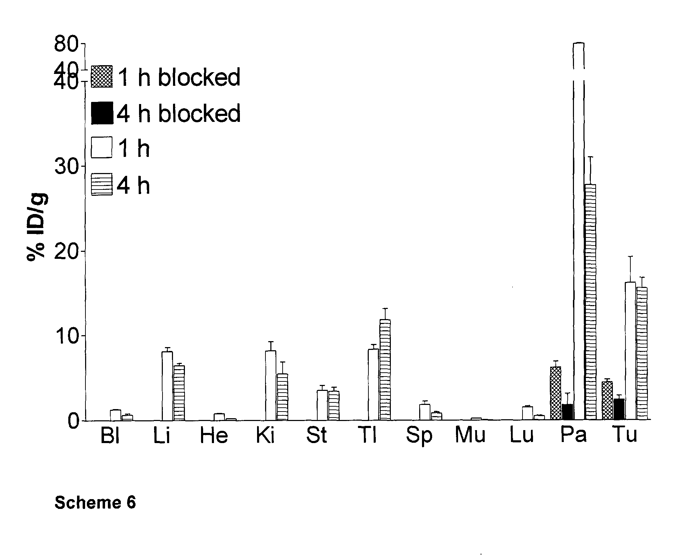

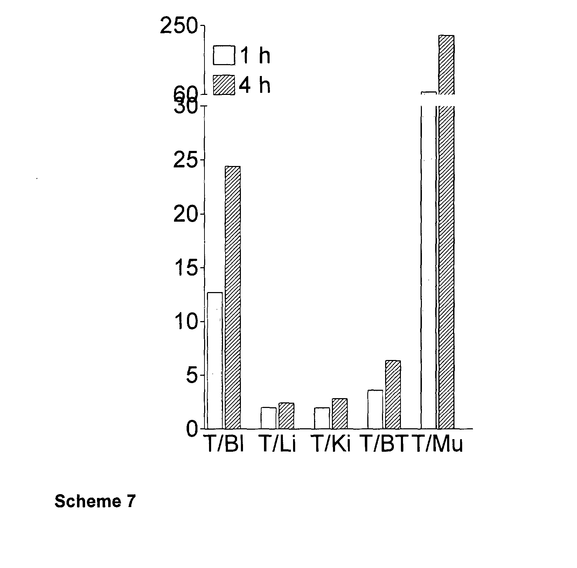

## Claims

1. A method for the synthesis, labeling with technetium-99m, the radiochemical, biological - preclinical evaluation of analogs of the bombesin (BBN) antagonist (D-Phe⁶,Leu-NHEt¹³,des-Met¹⁴)BBN(6-14), modified by 1,4,8,11-tetraazaundecane derivatives and labeled with technetium-99m and their application in Oncology.

2. A method for the synthesis in solution of analogs of the bombesin (BBN) antagonist (D-Phe⁶,Leu-NHEt¹³,des-Met¹⁴)BBN(6-14), modified by 1,4,8,11-tetraazaundecane derivatives according to claim 1, in which the respective BOC-protected 1,4,8,11-tetraazaundecane derivative was coupled to the terminal amino group of the peptide chain of the bombesin (BBN) antagonist (D-Phe⁶,Leu-NHEt¹³,des-Met¹⁴)BBN(6-14), either through a benzylamino diglycolic acid spacer (BBN-1) or directly, through a carboxy group (BBN-2). The above synthesis was performed in the presence of thiouronium salts in alkaline medium. The BOC-protected products after purification, were deprotected in acidic medium and purified by chromatography.

3. A method for the synthesis according to claim 2, in which the coupling of the 1,4,8,11-tetraazaundecane derivatives to the terminal amino group of the peptide chain of the bombesin (BBN) antagonist (D-Phe⁶,Leu-NHEt¹³,des-Met¹⁴)BBN(6-14), either through a spacer or directly, was performed in solution.

4. A method of labeling of the analogs derived according to claims 1-3 with radioactive technetium-99m, in which the binding of the radionuclide takes place in aqueous alkaline medium in the presence of citrate with tin chloride as the reducing agent.

5. A method of labeling with radioactive technetium-99m according to claim 4, in which the labeling yield as well as the radiochemical purity of the labeled product exceeds 95%.

6. A method of synthesis and labeling according to claims 1 - 5, which leads to hydrophilic and in vitro stable products.

7. A method of synthesis and labeling according to claims 1 - 6, which leads to with a high affinity and selectivity for the BBN/GRP receptors, fast clearance and excretion from the body through the kidneys and the urinary tract and stable in blood compounds, that do not exhibit non-specific accumulation in the rest of the body.

8. The application of the labeled with technetium-99m analogs of (D-Phe⁶,Leu-NHEt¹³,des-Met¹⁴)BBN (6-14) according to claim 1, in Oncology.
